# EUROPÄISCHE PATENTSCHRIFT

(11) **EP 3 708 103 B1**
(45) Veröffentlichungstag und Bekanntmachung des Hinweises auf die Patenterteilung: **30.06.2021**
(21) Anmeldenummer: 20161222.3
(22) Anmeldetag: 05.03.2020
(51) Int. Cl.: A61B 18/14, A61B 18/00

(54) **ELEKTRODENINSTRUMENT UND RESEKTOSKOP MIT GREIFFUNKTION**
ELECTRODE INSTRUMENT AND RESECTOSCOPE WITH GRIPPING FUNCTION
INSTRUMENT À ÉLECTRODES ET RÉSECTOSCOPE AYANT UNE FONCTION DE PRÉHENSION

(30) Priorität: 13.03.2019 DE 102019106430
(43) Veröffentlichungstag der Anmeldung: 16.09.2020
(73) Patentinhaber: Olympus Winter & Ibe GmbH, 22045 Hamburg (DE)
(72) Erfinder: BROCKMANN, Christian, 21279 Hollenstedt (DE); MIERSCH, Hannes, 22149 Hamburg (DE)
(74) Vertreter: Meissner Bolte Partnerschaft mbB

(56) Entgegenhaltungen:
- EP-A1- 1 974 683
- EP-A1- 3 437 581
- US-A- 5 196 011
- US-A1- 2014 171 824

## Beschreibung

Die Erfindung betrifft Elektrodeninstrumente zur Verwendung in einem Resektoskop der im Oberbegriff des Anspruchs 1 genannten Art sowie ein Resektoskop der im Oberbegriff des Anspruchs 9 genannten Art.

Elektrodeninstrumente und Resektoskope der gattungsgemäßen Art werden vor allem in der Urologie bei elektrochirurgischen Arbeiten in der Blase und der Urethra verwendet. Sie werden üblicherweise zur Resektion und Vaporisation von Gewebe, zum Beispiel von Gewebe im unteren Harntrakt, verwendet. Dazu umfassen die Resektoskope ein gattungsgemäßes Elektrodeninstrument, das innerhalb des Resektoskopschaftes längsverschiebbar gelagert ist und nach dem Einführen des Resektoskops mit seinem distalen Arbeitsende aus dem distalen Ende des Schaftes hervorgeschoben werden kann. Das Elektrodeninstrument umfasst an seinem distalen Arbeitsende eine elektrochirurgische Elektrode, zum Beispiel in Form eines Loops (Schlinge) oder Vaporisationsknopfes (z. B. PlasmaButtons). Solche Instrumente sind zum Beispiel die OES PRO Resektoskope (Olympus) oder andere Dauerspül-Resektoskope nach Iglesias wie beispielsweise in EP 3 437 581 A1 beschrieben.

Werden die Elektrodeninstrumente zur Resektion von Gewebe verwendet, so ist es oftmals wünschenswert oder notwendig, das abgetrennte Gewebe von der Eingriffsstelle zu entfernen, z. B. um eine weitere Ausbreitung einer Erkrankung, wie einer Tumorerkrankung, zu verhindern und/oder eine Gewebeprobe für diagnostische Zwecke zu entnehmen. Die Gewebeentnahme wird derzeit oftmals nicht mittels des Resektoskops sondern mit einem separat eingeführten Greifinstrument durchgeführt. Dieses Verfahren birgt jedoch die Risiken, dass bei sukzessiver Einführung der Instrumente Gewebefragmente verloren gehen können oder dass bei gleichzeitiger Einführung der Instrumente verschiedene Einführöffnungen genutzt werden müssen, sodass der Eingriff für den Patienten weniger schonend ist. Aus diesem Grund ist es wünschenswert, dasselbe Instrument für die Resektion und die Entnahme von Gewebe verwenden zu können.

Verschiedentlich wurden bestehende Resektoskope sowohl zur Geweberesektion als auch zur Entnahme des Gewebes verwendet. Dabei wurde nach der elektrochirurgischen Trennung eines Gewebefragments vom umliegenden Gewebe, z. B. mittels einer Schlingenelektrode, das Gewebefragment zwischen der Schlingenelektrode und dem im Resektoskopschaft angeordneten Innenrohr oder der Optik eingeklemmt. Anschließend wurden Elektrodeninstrument, Gewebefragment und Innenrohr gemeinsam aus dem Außenschaft (Hüllrohr) des Resektoskops und damit auch dem Patienten gezogen. Diese Vorgehensweise birgt jedoch die Gefahr einer Beschädigung der empfindlichen Elektrode. Darüber hinaus ist diese Greiffunktion von der Geometrie und Anordnung von Elektrode und Innenrohr abhängig, sodass bauliche Veränderungen an diesen Elementen zu einem Verlust oder einer Veränderung der Greiffunktion führen können.

Es besteht daher ein Bedarf an verbesserten Elektrodeninstrumenten und entsprechenden Resektoskopen für die elektrochirurgische Chirurgie mittels derer Gewebe sowohl durchtrennt, als auch aus dem Körper entfernt werden kann.

### Beschreibung

Gelöst wird diese Aufgabe durch ein Elektrodeninstrument mit den Merkmalen von Anspruch 1 und ein Resektoskop mit den Merkmalen von Anspruch 9. Erfindungsgemäß ist insbesondere vorgesehen, an mindestens einem der Tragarme des Elektrodeninstruments ein Gewebekontaktteil anzuordnen, zwischen dem und dem distalen Ende des Innenrohrs und/oder eines Durchgangsinstruments Gewebefragmente eingeklemmt oder an dem Gewebefragmente aufgespießt werden können. Die so an dem Elektrodeninstrument oder dem Resektoskop gehaltenen Gewebefragmente können dann dem Körper des Patienten entnommen werden.

In einem ersten Aspekt betrifft die Erfindung daher ein Elektrodeninstrument zur Verwendung in einem Resektoskop, wobei das Elektrodeninstrument einen langgestreckten Schaftabschnitt mit zwei Tragarmen aufweist, durch die hindurch sich ein Leiter erstreckt, der am distalen Ende des Elektrodeninstruments eine mit Hochfrequenzstrom beaufschlagbare Elektrode bildet, die zwischen den distalen Enden der Tragarme angeordnet ist, dadurch gekennzeichnet, dass mindestens einer der Tragarme in seinem distalen Endbereich zum Halten von Gewebefragmenten ein an der Außenwand des Tragarms angeordnetes, im Verhältnis zur Längsachse des Tragarms abgewinkeltes Gewebekontaktteil aufweist. Das erfindungsgemäße Gewebekontaktteil ist ausgebildet und angeordnet zum Halten von Gewebefragmenten zwischen dem Gewebekontaktteil und dem distalen Ende eines Innenrohrs und/oder dem distalen Ende eines Durchgangsinstruments eines Resektoskops. Dabei wird das Gewebefragment somit zwischen dem Gewebekontaktteil und einer proximal von dem Gewebekontaktteil angeordneten Komponente des Resektoskops eingeklemmt. Hierzu wird das Elektrodeninstrument zunächst nach distal derart verschoben, dass das Gewebekontaktteil distal von einem abgetrennten Gewebefragment angeordnet ist, und anschließend in proximale Richtung in den Resektoskopschaft derart zurückgezogen, dass das Gewebefragment zwischen der proximalen Oberfläche des Gewebekontaktteils und einer der oben genannten weiteren Komponenten des Resektoskops eingeklemmt wird.

In einem zweiten Aspekt betrifft die Erfindung ein Resektoskop für die endoskopische Chirurgie mit einem Hüllrohr, dadurch gekennzeichnet, dass innerhalb des Hüllrohres ein erfindungsgemäßes Elektrodeninstrument längsverschiebbar gelagert ist. Das erfindungsgemäße Resektoskop ist für verschiedene Eingriffe in der endoskopischen Chirurgie geeignet, insbesondere in der elektrochirurgischen Chirurgie. So kann das Resektoskop beispielsweise für die Prostata-Resektion verwendet werden. Gleichzeitig kann das Resektoskop aber auch für eine Vielzahl anderer Operationen eingesetzt werden, zum Beispiel für Blasen-Resektionen.

In üblicher Ausbildungsweise weist das erfindungsgemäße Resektoskop einen rohrartigen Schaft auf. Der Resektoskopschaft umfasst ein langgestrecktes Hüllrohr. Neben dem Schaft umfasst das Resektoskop zum Halten und Bedienen ein Griffsystem, das üblicherweise aus zwei Griffteilen besteht.

Das erfindungsgemäße Elektrodeninstrument wird als Durchgangsinstrument in einem solchen Resektoskop verwendet. Dazu ist das Elektrodeninstrument längsverschiebbar in dem Resektoskop gelagert, vorzugsweise innerhalb des Hüllrohres des Resektoskops. Das Elektrodeninstrument kann außerhalb eines in dem Hüllrohr verlaufenden Innenrohrs, d.h. zwischen Hüllrohr und Innenrohr, angeordnet sein oder innerhalb des Innenrohres. Zusätzlich zu dem Elektrodeninstrument können weitere Durchgangsinstrumente in dem Schaft des Resektoskops angeordnet sein. Üblicherweise umfasst das Resektoskop zusätzlich beispielsweise eine stabförmige Optik, ein Beleuchtungsmittel und/oder ein Spülsystem.

Das Elektrodeninstrument weist in üblicher Ausbildungsweise einen langgestreckten Schaftabschnitt auf, der im Inneren des Resektoskopschaftes, wie oben beschrieben, längsverschiebbar und optional um die Längsachse rotierbar angeordnet ist. Das proximale Ende des Schaftabschnitts ist funktional mit einem Schlitten des Transporteurs des Resektoskops verbunden, der mittels des Handgriffs betätigt werden kann. Am distalen Ende des Elektrodeninstruments ist eine mit Hochfrequenzstrom beaufschlagbare Elektrode ausgebildet.

Das Elektrodeninstrument weist, insbesondere im Schaftabschnitt, zwei Tragarme auf. Die Elektrode kann sicher zwischen den distalen Enden der beiden Tragarme gehalten werden, ohne die Sicht auf die Eingriffsstelle in erheblichem Maße zu beeinträchtigen. Die Tragarme können sich vom distalen Ende im Wesentlichen gerade bis zum proximalen Ende des Elektrodeninstruments erstrecken. Alternativ können die Tragarme als Gabelrohre ausgebildet und proximal der Elektrode in einer Gabelung zusammengeführt sein. Diese Anordnung hat sich in einigen Resektoskopen als besonders platzsparend und stabil erwiesen. Fachleuten sind weitere geeignete Ausführungen des Schaftabschnitts geläufig. So ist es beispielsweise auch möglich, die Tragarme proximal der Elektrode in einen rohrförmigen Elektrodenschaft übergehen zu lassen. Auf diese Weise könnte das Elektrodeninstrument gleichzeitig als Innenrohr verwendet werden. Erfindungsgemäß ist es somit nur erforderlich, dass das Elektrodeninstrument in seinem distalen Endbereich zwei Tragarme aufweist, zwischen denen die Elektrode gehalten werden kann.

Durch die Tragarme bzw. den Schaftabschnitt hindurch erstreckt sich ein Leiter, der am distalen Ende des Elektrodeninstruments eine mit Hochfrequenzstrom beaufschlagbare Elektrode bildet. Dieser Leiter ist elektrisch leitfähig und kann beispielsweise ein Leiterdraht sein. Der Leiter ist im Bereich der Tragarme nach außen, z. B. gegenüber dem Resektoskopschaft elektrisch isoliert. Zu diesem Zweck umfassen die Tragarme in der Regel eine den Leiter umgebene Isolierhülle, d.h. eine elektrisch isolierende Hülle. Die Isolierhülle kann eine Hohlzylinderform aufweisen, in deren Inneren der Leiter verläuft. Am proximalen Ende der Tragarme ist der Leiter über den proximalen Abschnitt des Resektoskops mit einer Stromquelle verbunden.

Die von dem Leiter gebildete Elektrode kann unterschiedliche Formen aufweisen, wird jedoch jeweils zwischen den distalen Enden der beiden Tragarme getragen. Die Elektrode kann beispielsweise eine Schlingenelektrode oder ein Vaporiationsknopf sein, wobei erstere Ausführung bevorzugt ist.

Erfindungsgemäß weist mindestens einer der Tragarme in seinem distalen Endbereich zum Halten von Gewebefragmenten ein an der Außenwand des Tragarms angeordnetes, im Verhältnis zur Längsachse des Tragarms abgewinkeltes Gewebekontaktteil auf. Das an einem Tragarm angeordnete Gewebekontaktteil ermöglicht das sichere Halten von Gewebefragmenten, ohne die endständige Elektrode belasten zu müssen, sodass die Gewebefragmente auf sichere und einfache Weise von der Eingriffsstelle entfernt werden können. Die gehaltenen Gewebefragmente werden in der Regel gemeinsam mit einem Innenschaft und/oder dem Elektrodeninstrument aus dem Hüllrohr des Resektoskops herausgezogen werden, sodass die Gewebefragmente vorzugsweise einen maximalen Durchmesser aufweisen, der kleiner ist als der Innendurchmesser des Hüllrohres. Es ist aber auch denkbar, das Resektoskop inklusive eines gehaltenen Gewebefragments vollständig von der Eingriffsstelle zu entfernen, sodass auch größere Gewebefragmente dem Körper eines Patienten entnommen werden können.

Wie bereits ausgeführt ist das erfindungsgemäße Gewebekontaktteil ausgebildet und angeordnet zum Halten von Gewebefragmenten zwischen dem Gewebekontaktteil und dem distalen Ende eines Innenrohrs und/oder dem distalen Ende eines Durchgangsinstruments eines Resektoskops. Hierzu wird das Elektrodeninstrument zunächst nach distal derart verschoben, dass das Gewebekontaktteil distal von einem abgetrennten Gewebefragment angeordnet ist, und anschließend in proximale Richtung in den Resektoskopschaft derart zurückgezogen, dass das Gewebefragment zwischen der proximalen Oberfläche des Gewebekontaktteils und dem distalen Ende eines Innenrohrs und/oder eines Durchgangsinstruments, wie einer Optik eingeklemmt wird. Hierbei ist es ausreichend, das Gewebefragment an einer Seite des Innenrohrs abzustützen. Es ist nicht erforderlich, dass das Gewebefragment das distale Ende des Innenrohres vollständig verschließt.

Alternativ oder zusätzlich kann das Gewebekontaktteil zum Einstechen in Gewebefragmente ausgebildet und angeordnet sein. In dieser Ausführungsform ist es zum Halten des Gewebefragments nicht zwingend erforderlich, das proximale Ende des Fragments an einer weiteren Komponente des Resektoskops abzustützen. Stattdessen ist das Gewebekontaktteil ausreichend lang, um ein aufgespießtes Gewebefragment sicher zu halten. Das zum Einstechen in Gewebefragmente ausgebildete Gewebekontaktteil ist vorzugsweise in mindestens einer Dimension flach ausgebildet, um ein einfaches Aufspießen des Gewebefragments zu ermöglichen. Vorzugsweise ist das zum Einstechen ausgebildete Gewebekontaktteil stabförmig ausgebildet. Wie weiter unten noch näher erläutert, kann das stabförmige oder anders geformte Gewebekontaktteil dabei gebogen bzw. gekrümmt sein, um den Dimensionen des Resektoskopschaftes angepasst zu werden.

Es ist auch denkbar, das Gewebekontaktteil mit einer spitz zulaufenden Spitze auszubilden. Hierdurch wird sichergestellt, dass ein Gewebefragment schnell und sicher eingestochen, aufgenommen und auf das Gewebekontaktteil aufgeschoben werden kann ohne bei diesen Schritten auseinanderzufallen.

Erfindungsgemäß weist mindestens einer der Tragarme ein solches Gewebekontaktteil auf. Es ist denkbar, dass an beiden Tragarmen Gewebekontaktteile angeordnet sind. Dies ist jedoch erfindungsgemäß nicht erforderlich. Es kann vorteilhaft sein, nur an einem der Tragarme ein Gewebekontaktteil anzuordnen. Auf diese Weise kann beispielsweise Material und Platz gespart werden.

Das Gewebekontaktteil ist im distalen Endbereich des Tragarmes angeordnet. Das Gewebekontaktteil sollte in einem Resektoskop distal von dem distalen Ende des Innenrohres und/oder Durchgangsinstruments angeordnet sein. So wird nicht nur sichergestellt, dass dieselben als Klemmpartner zur Verfügung stehen, sondern auch, dass ausreichend Platz für das Gewebefragment im Inneren des Hüllrohres zur Verfügung steht. Das Gewebekontaktteil kann im Resektoskop bei maximal nach proximal verschobenem Elektrodeninstrument beispielsweise im Bereich der Isolierspitze des Resektoskops angeordnet sein. Üblicherweise weisen Resektoskope an ihrem distalen Ende eine solche elektrisch isolierende Spitze auf, um Kurzschlüsse zwischen der Elektrode und den elektrisch leitfähigen Komponenten des Resektoskops zu verhindern.

Das Gewebekontaktteil kann in einem Abschnitt des Tragarms angeordnet sein, der von einer elektrisch isolierenden Isolierhülle ummantelt ist. Auch das Gewebekontaktteil kann aus einem elektrisch isolierenden Material sein. Dies ist besonders vorteilhaft, da so kein Isolierabstand zwischen dem Gewebekontaktteil und der Elektrode eingehalten werden muss. Geeignete elektrisch isolierende Materialien sind Fachleuten bekannt und umfassen beispielsweise Keramiken und Kunststoffe, insbesondere thermostabile Kunststoffe. Alternativ kann das Gewebekontaktteil aber auch in einem Abschnitt des Tragarms angeordnet sein, der nicht von einer elektrisch isolierenden Isolierhülle ummantelt ist. Dann könnte das Gewebekontaktteil z. B. unmittelbar am Leiter angeordnet sein. Auch in dieser Ausführungsform kann das Gewebekontaktteil aus einem elektrisch isolierenden Material sein.

Das Gewebekontaktteil ist jeweils an der Außenwand des Tragarms angeordnet und im Verhältnis zur Längsachse des Tragarms abgewinkelt. So kann die Längsachse des Gewebekontaktteils beispielsweise quer oder im Wesentlichen quer zur Längsachse des Tragarms verlaufen. Die Längsachse des Gewebekontaktteils bildet zur Längsachse des Tragarms jedoch vorzugsweise mindestens einen Winkel von 140° bis 40°, stärker bevorzugt einen Winkel von 120° bis 60°, beispielsweise etwa 90°. Es versteht sich, dass das Gewebekontaktteil diesen Winkel nicht mit seiner gesamten Länge bilden muss. Stattdessen bildet die Längsachse des Gewebekontaktteils die genannten Winkel mindestens abschnittsweise zur Längsachse des Tragarms, wobei bevorzugt mindestens ein mit dem Tragarm unmittelbar verbundener Abschnitt den genannten Winkel bildet.

Wie oben bereits angedeutet, kann die Form des Gewebekontaktteils an eine Innenform des Resektoskops angepasst sein, d.h. teilweise formkomplementär zu dieser ausgebildet sein, z. B. zur Innenwand des Hüllrohres oder des Innenrohres. So kann sich das Gewebekontaktteil beispielsweise ausgehend von dem Tragarm teilkreisförmig gebogen von dem Tragarm weg erstrecken. Die Krümmung des Gewebekontaktteils kann dabei z. B. der Krümmung einer Schlingenelektrode entsprechen. Bei derart gekrümmten Gewebekontaktteilen ist es bevorzugt, dass das Gewebekontaktteil nicht direkt proximal von (d.h. von distal aus gesehen: hinter) der Schlingenelektrode angeordnet ist, sondern auf der Seite des Tragarms angeordnet ist, welche von der Seite abgewandt ist, in die sich die Schlinge der Schlingenelektrode erstreckt.

Erfindungsgemäß weist das zum Einstechen ausgebildete Gewebekontaktteil eine Länge auf und das zum Einklemmen ausgebildete Gewebekontaktteil eine proximale Fläche auf, die lang bzw. groß genug sind, um das Gewebefragment sicher zu halten. Insbesondere sollte das Gewebekontaktteil bei dem Herausziehen des Innenrohres und/oder Elektrodeninstruments aus dem Hüllrohr nicht von dem Gewebekontaktteil abrutschen. Um ein Abrutschen von dem zum Einstechen ausgebildeten Gewebekontaktteil auszuschließen, weist das Gewebekontaktteil zum Beispiel eine Länge auf, die mindestens 1/8 der Länge der Elektrode des Instruments ist, vorzugsweise mindestens 1/4, stärker bevorzugt mindestens 1/2. Das Gewebekontaktteil kann sich ausgehend von dem Tragarm mindestens bis in eine Sagittalebene des Elektrodeninstruments erstrecken, die sich ausgehend von dem Tragarm auf 1/8 der Entfernung zum zweiten Tragarm erstreckt, vorzugsweise 1/4 der Entfernung. Als Sagittalebenen werden alle Ebenen bezeichnet, die sich durch das Elektrodeninstrument von oben nach unten und proximal nach distal erstrecken, wobei die Angaben "oben" und "unten" sich auf eine Haltung des Instruments beziehen, in der es normalerweise verwendet wird, und in der beispielsweise der Handgriff des Resektoskops nach unten weist. Die Sagittalebene in der Mitte zwischen beiden Tragarmen, d.h. die Sagittalebene, die sich ausgehend von dem Tragarm auf 1/2 der Entfernung zum zweiten Tragarm erstreckt, kann auch als Medianebene bezeichnet werden.

Um das Halten des Gewebefragments zwischen dem Gewebekontaktteil und einer weiteren Komponente besonders sicher zu gestalten, kann das Gewebekontaktteil mindestens abschnittsweise distal von einem in dem Hüllrohr angeordneten Innenrohr und/oder einem in dem Hüllrohr angeordneten Durchgangsinstrument angeordnet sein. In diesem Zusammenhang bezeichnet der Begriff "distal" eine Anordnung in der das Gewebekontaktteil in gerader, axialer Verlängerung der entsprechenden Komponente angeordnet ist.

### Kurze Beschreibung der Figuren

In den Zeichnungen sind Ausführungsbeispiele der Erfindung schematisch dargestellt. Es zeigen:
- **Fig. 1**: eine schematische, seitliche Schnittdarstellung eines erfindungsgemäßen Resektoskops;
- **Fig. 2**: eine schematische, seitliche Schnittdarstellung eines erfindungsgemäßen Elektrodeninstruments;
- **Fig. 3**: eine schematische, seitliche Schnittdarstellung des distalen Endbereichs eines erfindungsgemäßen Resektoskops;
- **Fig. 4**: eine schematische Frontalansicht des in Fig. 3 gezeigten Endbereichs (links) und eine schematische Schnittansicht entlang der Achse "LG" in Fig. 3 (rechts);
- **Fig. 5**: eine schematische, seitliche Schnittdarstellung des in Fig. 3 dargestellten Endbereichs, wobei ein Gewebefragment auf dem Gewebekontaktteil gehalten ist;
- **Fig. 6**: eine schematische, seitliche Schnittdarstellung des distalen Endbereichs eines alternativen erfindungsgemäßen Resektoskops;
- **Fig. 7**: eine schematische Frontalansicht des in Fig. 6 gezeigten Endbereichs;
- **Fig. 8**: eine schematische Draufsicht auf das Elektrodeninstrument, das in dem Resektoskop von Fig. 6 verwendet ist;
- **Fig. 9**: eine schematische, seitliche Schnittdarstellung des in Fig. 6 dargestellten Endbereichs, wobei ein Gewebefragment zwischen dem Gewebekontaktteil und einer Optik gehalten ist; und
- **Fig. 10**: eine schematische, perspektivische Sicht ins Innere des in Fig. 6 gezeigten Endbereichs.

### Ausführungsbeispiele

Weitere Vorteile, Kennzeichen und Merkmale der vorliegenden Erfindung werden bei der nachfolgenden detaillierten Beschreibung von Ausführungsbeispielen anhand der beigefügten Zeichnungen deutlich. Allerdings ist die Erfindung nicht auf diese Ausführungsbeispiele beschränkt.

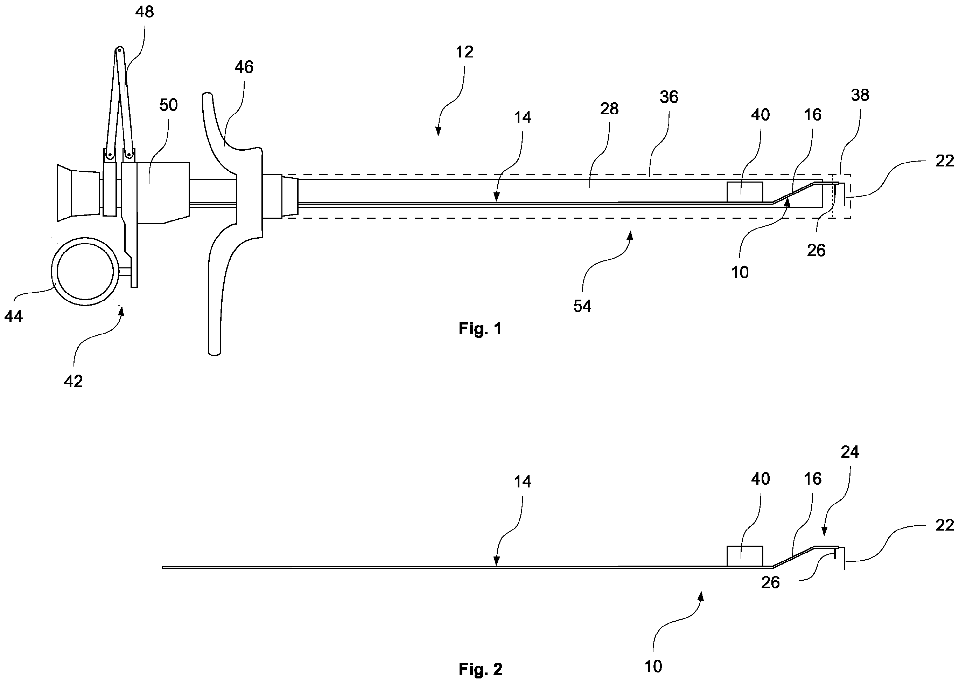

Fig. 1 zeigt eine Schnittdarstellung eines erfindungsgemäßen Resektoskops 12, das ein erfindungsgemäßes Elektrodeninstrument 10 umfasst. Fig. 2 zeigt das Elektrodeninstrument 10 getrennt von dem Resektoskop 12. In üblicher Ausbildungsweise umfasst das Resektoskop 12 einen Handgriff 42 und einen Schaft 54, der an seinem distalen Ende eine Isolierspitze 38 aufweist. Der Handgriff 42 ist zum Halten des Resektoskops 12 in einer Hand und zur, vorzugsweise einhändigen, Betätigung der durch den Schaft 54 hindurch verlaufenden Durchgangsinstrumente ausgebildet.

Das dargestellte Resektoskop 12 weist einen passiven Transporteur auf, bei dem der Schlitten 50 durch Relativbewegung der proximal von dem Schaft 54 angeordneten Griffteile 44 und 46 zueinander gegen eine von einer Federbrücke 48 aufgebrachte Federkraft in distale Richtung gegen das distale, erste Griffteil 46 verschoben wird. Bei der Verschiebung des Schlittens 50 in distale Richtung gegen das Griffteil 46 wird das Elektrodeninstrument 10 in nicht dargestellter Weise zwangsgeführt nach distal verschoben. Bei einer Entlastung der Griffteile 44, 46 zwingt die von der Federbrücke 48 erzeugte Federkraft den Schlitten 50 zurück in seine Ruheposition, wobei das Elektrodeninstrument 10 in proximale Richtung gezogen wird. Bei der Rückverschiebung des Schlittens 50 kann ohne Handkraft des Operateurs, also passiv, ein elektrochirurgischer Eingriff mit dem Elektrodeninstrument 10 vorgenommen werden.

Der Schaft 54 des Resektoskops 12 umfasst ein Hüllrohr 36, in dessen Inneren mehrere längliche Durchgangsinstrumente verlaufen, insbesondere eine hier nicht dargestellte Optik und das Elektrodeninstrument 10. Die Optik sowie weitere Durchgangsinstrumente können in dem Innenrohr 28 verlaufen, das zwischen den Tragarmen 16, 18 des Elektrodeninstruments 10 in dem Hüllrohr 36 angeordnet ist. Das Elektrodeninstrument 10 ist dabei zur Stabilisierung in seinem Schaftabschnitt 14 durch ein Halteelement 40 gegen eine radiale Verschiebung gesichert. Das Halteelement 40 weist einen teilkreisförmigen Querschnitt auf, der in dem vorliegenden Instrument an der Außenwand des Innenrohrs 28 anliegt. Es ist auch denkbar, ein Halteelement 40 an anderen Elementen des Schaftes 54 abzustützen, beispielsweise an der Innenwand des Hüllrohres 36. Der Querschnitt des Halteelements 40 ist im vorliegenden Fall in etwa halbkreisförmig, so dass sich das Halteelement 40 formschlüssig an die Außenwand des Innenrohres 28 anlegt und das Elektrodeninstrument 10 weiterhin axial verschiebbar ist. Mit anderen Worten ist das Halteelement 40 teilweise formkomplementär zu der Außenwand des Innenrohres 28.

An seinem distalen Ende weist das Elektrodeninstrument 10 eine Elektrode 22 auf, die im vorliegenden Fall als Schlingenelektrode bzw. Schneidschlinge ausgebildet ist. Das Instrument ist als bipolares Instrument ausgebildet und mit einer nicht dargestellten Gegenelektrode versehen. Mittels der Schlingenelektrode ist das medizinische Fachpersonal in der Lage, bei einem chirurgischen Eingriff Gewebe von der Eingriffsstelle abzutragen.

Darüber hinaus weist das Elektrodeninstrument an einem Tragarm 16 ein Gewebekontaktteil 26 auf. Das Gewebekontaktteil 26 ist im distalen Endbereich 24 des Tragarms 16 an dessen Außenwand angeordnet. Die Elektrode 22 ist distal beabstandet am distalen Ende des Tragarms 16 gehalten. In der gezeigten Ausführungsform erstreckt sich das Gewebekontaktteil 26 ausgehend von dem Tragarm 16 nach radial innen, d.h. in Richtung des Mittelpunkts des Hüllrohres 36.

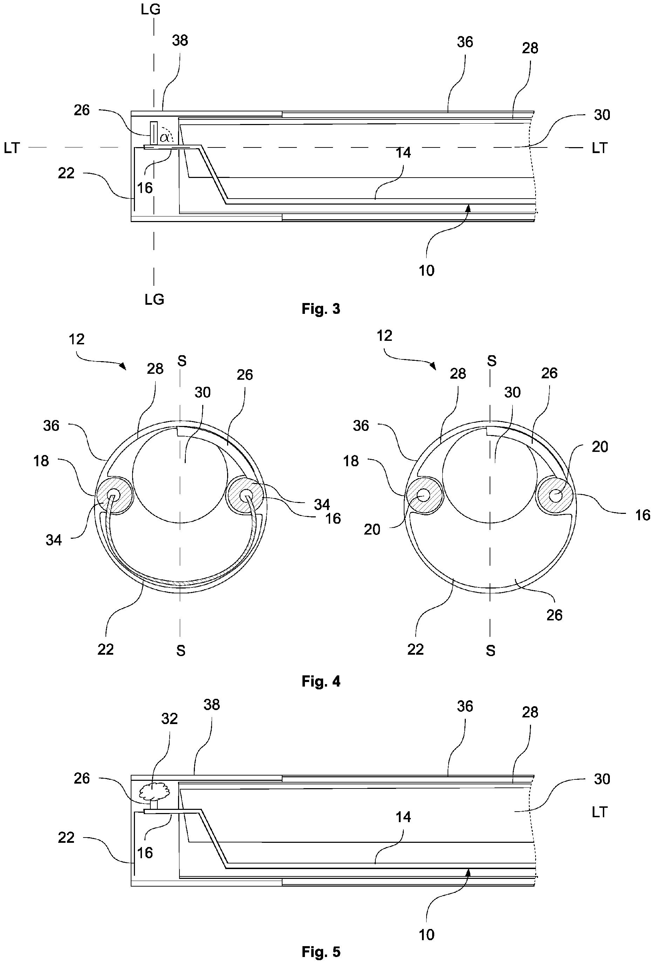

Fig. 3 zeigt eine detailliertere, schematische, seitliche Schnittdarstellung des distalen Endbereichs einer Ausführungsform des erfindungsgemäßen Resektoskops 12. Das Elektrodeninstrument 10 weist ein Gewebekontaktteil 26 auf, das sich ausgehend von dem Tragarm 16 in entgegengesetzter Weise erstreckt, wie das in den Fig. 1 und 2 gezeigte. Das Gewebekontaktteil 26, insbesondere dessen Längsachse LG, bildet dabei zum Tragarm 16, insbesondere dessen Längsachse LT, einen Winkel α von 90°. Zur Erhöhung der Übersichtlichkeit ist der Tragarm 16 hier dünner dargestellt als in den folgenden Figuren. Das Elektrodeninstrument 10 ist in der gezeigten Stellung maximal nach proximal verschoben und damit vollständig in das Hüllrohr 36 zurückgezogen. In dieser Stellung sind sowohl die Elektrode 22 als auch das Gewebekontaktteil 26 in dem Bereich des Schaftes 54 angeordnet, in dem die Isolierspitze 38 das distale Ende des Schaftes 54 bildet. Gleichzeitig ist das Gewebekontaktteil 26 distal von dem distalen Ende des Innenrohres 28 und des Durchgangsinstruments 30, das hier eine stabförmige Optik ist, angeordnet.

Das Gewebekontaktteil 26 weist die Form eines gekrümmten Stabes auf, wie insbesondere auch in Fig. 4 erkennbar ist, und ist dadurch zum Einstechen in ein Gewebefragment geeignet. Fig. 4 zeigt eine schematische Frontalansicht des in Fig. 3 gezeigten Endbereichs (links) und eine schematische Schnittansicht entlang der Achse "LG" in Fig. 3 (rechts). Die Tragarme 16 und 18 durchlaufen den Schaft des Resektoskops 12 zwischen dem Hüllrohr 36 und dem Innenrohr 28. Im Inneren des Innenrohrs 28 ist ein Durchgangsinstrument 30 angeordnet, z. B. eine Optik. Am distalen Ende der Tragarme 16, 18 ist zwischen den Tragarmen 16, 18 eine Elektrode 22 in Schlingenform ausgebildet. Beide Tragarme 16, 18 weisen eine Isolierhülle 34 auf, die aus einem elektrisch isolierenden Material besteht. Innerhalb der Isolierhülle 34 ist ein Leiter 20 in Drahtform ausgebildet, der am distalen Ende der Tragarme 16, 18 die Schlingenelektrode bildet.

Das Gewebekontaktteil 26 ist an dem Tragarm 16 angeordnet und proximal von der am Tragarm angeordneten Elektrode 22 distanziert. Dabei erstreckt sich das Gewebekontaktteil 26 ausgehend von dem Tragarm 16 in etwa in die entgegengesetzte Richtung relativ zu der Richtung in die sich die Elektrode 22 ausgehend von dem Tragarm 16 erstreckt. Auf diese Weise behindert die Anordnung des Gewebekontaktteils 26 die Verwendung der Elektrode 22 nicht. Die Länge des Gewebekontaktteils 26 entspricht in etwa der Hälfte der Länge der Elektrode 22, wobei die Krümmung der beiden Elemente in etwa identisch ist. Dabei ist das Gewebekontaktteil 26 etwas dicker als die Elektrode 22 ausgebildet.

Fig. 5 zeigt eine schematische, seitliche Schnittdarstellung des in Fig. 3 dargestellten Endbereichs, wobei ein Gewebefragment 32 auf dem Gewebekontaktteil gehalten ist. Das Gewebefragment 32 wurde zunächst mittels der Elektrode 22 von dem umliegenden Gewebe des Patienten getrennt. Anschließend wurde das Gewebekontaktteil 26 in das Gewebefragment 32 eingestochen. Da das Gewebefragment 32 eine Größe aufweist, die an keiner Stelle den Innendurchmesser des Hüllrohres 36 überschreitet, konnte das Gewebefragment 32 anschließend gemeinsam mit dem Elektrodeninstrument 10 durch axial-proximale Verschiebung des Elektrodeninstruments 10 in das Hüllrohr 36 zurückgezogen werden.

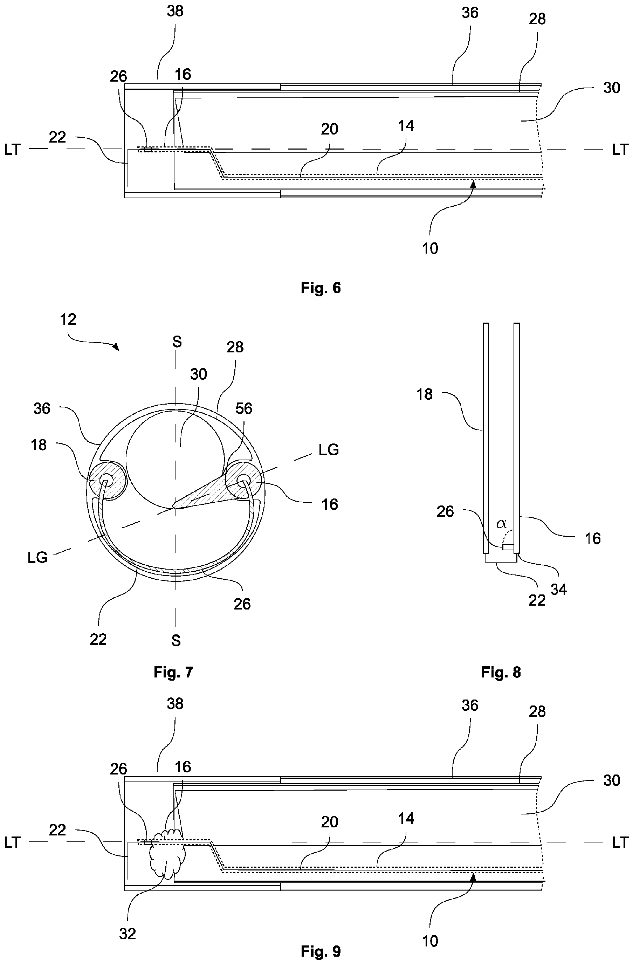

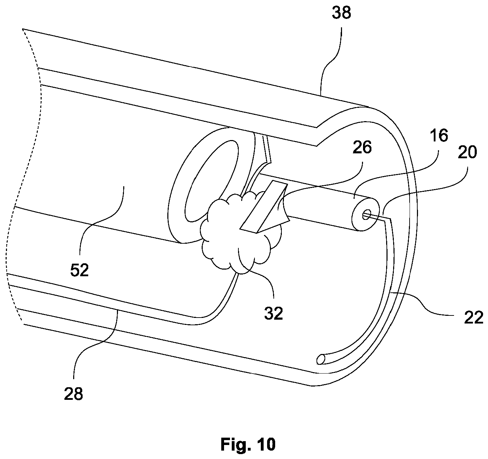

Fig. 6 zeigt eine schematische, seitliche Schnittdarstellung des distalen Endbereichs eines alternativen erfindungsgemäßen Resektoskops 12. Im Unterschied zu dem in Fig. 3 bis 5 gezeigten Elektrodeninstrument 10, weist das hier dargestellte Elektrodeninstrument 10 ein Gewebekontaktteil 26 auf, das vorrangig zum Halten von Gewebefragmenten 32 zwischen dem Gewebekontaktteil 26 und einer weiteren Komponente des Resektoskops 12 gedacht ist, d.h. nicht unbedingt zum Einstechen in ein Gewebefragment 32, wobei letztes nicht ausgeschlossen ist. Die Fig. 6 bis 10 zeigen schematisch dieselbe Ausführungsform, wobei die Größenverhältnisse zwischen den Resektoskopkomponenten zur besseren Sichtbarkeit variieren. Fig. 7 zeigt eine schematische Frontalansicht des in Fig. 6 gezeigten Endbereichs während Fig. 8 eine schematische Draufsicht auf das Elektrodeninstrument, das in dem Resektoskop von Fig. 6 verwendet ist, darstellt. Fig. 9 zeigt eine schematische, seitliche Schnittdarstellung des in Fig. 6 dargestellten Endbereichs, wobei ein Gewebefragment 32 zwischen dem Gewebekontaktteil 26 und einer Optik 52 gehalten ist. Fig. 10 zeigt eine schematische, perspektivische Sicht ins Innere des in Fig. 6 gezeigten Endbereichs.

Das Gewebekontaktteil 26 erstreckt sich in der Ausführungsform der Fig. 6 bis 10, ausgehend von dem Tragarm 16, schnabel- oder dreiecksförmig in Richtung des Mittelpunkts des Hüllrohres 36. Das Ende des Gewebekontaktteils 26 erreicht dabei die Saggitalebene S zwischen den Tragarmen, welche die Medianebene zwischen den beiden Armen ist. Eine direkte Verbindung zu dem zweiten Tragarm 18 besteht nicht. Die unmittelbar an die Außenwand der Isolierhülle des Tragarms 16 angrenzende Verbindungsfläche 56 des Gewebekontaktteils 26 weist eine konkave Form auf, die sich an die runde Oberfläche des Tragarms 16 anschmiegen kann. Diese Verbindungsfläche 56 grenzt auf 20° oder mehr, vorzugsweise auf 35° oder mehr von dessen Umfang an den Tragarm an. Alternativ kann das Gewebekontaktteil 26 einen rohrförmigen Abschnitt aufweisen, der den Tragarm 16 formschlüssig umschließt. Auf diese Weise wird eine besonders sichere Verbindung zwischen Gewebekontaktteil 26 und Tragarm 16 gewährleistet.

Die in Fig. 8 dargestellte Draufsicht auf das Elektrodeninstrument 10 zeigt, dass die Tragarme 16 und 18 bis zum proximalen Ende des Elektrodeninstruments 10 im Wesentlichen parallel zueinander verlaufen. Beide Tragarme 16, 18 weisen einen Leiter 20 auf, der in Fig. 6 erkennbar ist, in der die Isolierhülle 34 gestrichelt dargestellt ist. Der Leiter 20 durchläuft beide Tragarme 16, 18 und bildet am distalen Ende der Tragarme 16, 18 eine Elektrode 22. Das Gewebekontaktteil 26 bildet auch hier zum Tragarm 16 einen Winkel α von etwa 90°. Es ist in weiteren Ausführungsformen angedacht, das Gewebekontaktteil 26 gekrümmt, zum Beispiel nach proximal gekrümmt, auszubilden.

## Patentansprüche

1. Elektrodeninstrument (10) zur Verwendung in einem Resektoskop (12), wobei das Elektrodeninstrument (10) einen langgestreckten Schaftabschnitt (14) mit zwei Tragarmen (16, 18) aufweist, durch die hindurch sich ein Leiter (20) erstreckt, der am distalen Ende des Elektrodeninstruments (10) eine mit Hochfrequenzstrom beaufschlagbare Elektrode (22) bildet, die zwischen den distalen Enden der Tragarme (16, 18) angeordnet ist, **dadurch gekennzeichnet, dass** mindestens einer der Tragarme (16, 18) in seinem distalen Endbereich (24) zum Halten von Gewebefragmenten ein an der Außenwand des Tragarms (16, 18) angeordnetes, im Verhältnis zur Längsachse (LT) des Tragarms (16, 18) abgewinkeltes Gewebekontaktteil (26) aufweist, wobei das Gewebekontaktteil (26) zum Halten von Gewebefragmenten (32) zwischen dem Gewebekontaktteil (26) und dem distalen Ende eines Innenrohrs (28) und/oder dem distalen Ende eines Durchgangsinstruments (30) eines Resektoskops (12) ausgebildet und angeordnet ist.

2. Elektrodeninstrument (10) nach Anspruch 1, **dadurch gekennzeichnet, dass** die Längsachse (LG) des Gewebekontaktteils (26) zur Längsachse (LT) des Tragarms (16, 18) mindestens abschnittsweise einen Winkel (a) von 140° bis 40° bildet.

3. Elektrodeninstrument (10) nach einem der Ansprüche 1 oder 2, **dadurch gekennzeichnet, dass** die Längsachse (LG) des Gewebekontaktteils (26) zur Längsachse (LT) des Tragarms (16, 18) einen Winkel (a) von 120° bis 60° bildet.

4. Elektrodeninstrument (10) nach einem der vorangegangenen Ansprüche, **dadurch gekennzeichnet, dass** das Gewebekontaktteil (26) aus einem elektrisch isolierenden Material ist.

5. Elektrodeninstrument (10) nach einem der vorangegangenen Ansprüche, **dadurch gekennzeichnet, dass** das Gewebekontaktteil (26) in einem Abschnitt des Tragarms (16, 18) angeordnet ist, der von einer elektrisch isolierenden Isolierhülle (34) ummantelt ist.

6. Elektrodeninstrument (10) nach einem der vorangegangenen Ansprüche, **dadurch gekennzeichnet, dass** sich das Gewebekontaktteil (26) ausgehend von dem Tragarm (16, 18) teilkreisförmig gebogen von dem Tragarm (16, 18) weg erstreckt.

7. Elektrodeninstrument (10) nach einem der vorangegangenen Ansprüche, **dadurch gekennzeichnet, dass** sich das Gewebekontaktteil (26) ausgehend von dem Tragarm (16, 18) mindestens bis in eine Sagittalebene (S) des Elektrodeninstruments (10) erstreckt, die sich ausgehend von dem Tragarm (16, 18) auf 1/8 der Entfernung zum zweiten Tragarm (18, 16) erstreckt, vorzugsweise auf 1/4 der Entfernung.

8. Elektrodeninstrument (10) nach einem der vorangegangenen Ansprüche, **dadurch gekennzeichnet, dass** das Gewebekontaktteil (26) zum Einstechen in Gewebefragmente (32) ausgebildet und angeordnet ist.

9. Resektoskop (12) für die endoskopische Chirurgie mit einem Hüllrohr (36), **dadurch gekennzeichnet, dass** innerhalb des Hüllrohres (36) ein Elektrodeninstrument (10) nach einem der Ansprüche 1 bis 8 längsverschiebbar gelagert ist.

10. Resektoskop (12) nach Anspruch 9, **dadurch gekennzeichnet, dass** das Gewebekontaktteil (26) mindestens abschnittsweise distal von einem in dem Hüllrohr (36) angeordneten Innenrohr (28) und/oder einem in dem Hüllrohr angeordneten Durchgangsinstrument (30) angeordnet ist.

11. Resektoskop (12) nach Anspruch 9, **dadurch gekennzeichnet, dass** das Gewebekontaktteil (26) mindestens abschnittsweise proximal von der Elektrode angeordnet ist.

## Claims

1. Electrode instrument (10) for use in a resectoscope (12), the electrode instrument (10) having an elongate shaft portion (14) with two support arms (16, 18) through which a conductor (20) extends which, at the distal end of the electrode instrument (10), forms an electrode (22) that can be subjected to high-frequency current and that is arranged between the distal ends of the support arms (16, 18), **characterized in that** at least one of the support arms (16, 18) has, in its distal end region (24), for holding tissue fragments, a tissue contact part (26) which is arranged on the outer wall of the support arm (16, 18) and is angled in relation to the longitudinal axis (LT) of the support arm 16, 18), the tissue contact part (26) being designed and arranged for holding tissue fragments (32) between the tissue contact part (26) and the distal end of an inner tube (28) and/or the distal end of a pass-through instrument (30) of a resectoscope (12) .

2. Electrode instrument (10) according to Claim 1, **characterized in that** the longitudinal axis (LG) of the tissue contact part (26) forms at least in part an angle (a) of 140° to 40° to the longitudinal axis (LT) of the support arm (16, 18).

3. Electrode instrument (10) according to either of Claims 1 and 2, **characterized in that** the longitudinal axis (LG) of the tissue contact part (26) forms an angle (a) of 120° to 60° to the longitudinal axis (LT) of the support arm (16, 18).

4. Electrode instrument (10) according to one of the preceding claims, **characterized in that** the tissue contact part (26) is made of an electrically insulating material.

5. Electrode instrument (10) according to one of the preceding claims, **characterized in that** the tissue contact part (26) is arranged in a portion of the support arm (16, 18) that is encased by an electrically insulating insulation sleeve (34).

6. Electrode instrument (10) according to one of the preceding claims, **characterized in that,** starting from the support arm (16, 18), the tissue contact part (26) extends away from the support arm (16, 18) in the shape of an arc of a circle.

7. Electrode instrument (10) according to one of the preceding claims, **characterized in that,** starting from the support arm (16, 18), the tissue contact part (26) extends at least into a sagittal plane (S) of the electrode instrument (10) which, starting from the support arm (16, 18), extends by 1/8 of the distance to the second support arm (16, 18), preferably by 1/4 of the distance.

8. Electrode instrument (10) according to one of the preceding claims, **characterized in that** the tissue contact part (26) is designed and arranged to pierce into tissue fragments (32).

9. Resectoscope (12) for endoscopic surgery, having an outer tube (36), **characterized in that** an electrode instrument (10) according to one of Claims 1 to 8 is mounted in a longitudinally displaceable manner within the outer tube (36).

10. Resectoscope (12) according to Claim 9, **characterized in that** the tissue contact part (26) is at least in part arranged distally from an inner tube (28) arranged in the outer tube (36) and/or from a pass-through instrument (30) arranged in the outer tube.

11. Resectoscope (12) according to Claim 9, **characterized in that** the tissue contact part (26) is at least in part arranged proximally from the electrode.

## Revendications

1. Instrument à électrode (10) pour l'utilisation dans un résectoscope (12), l'instrument à électrode (10) présentant une portion de tige allongée (14) avec deux bras de support (16, 18), à travers lesquels s'étend un conducteur (20) qui forme, à l'extrémité distale de l'instrument à électrode (10), une électrode (22) pouvant être sollicitée avec un courant à haute fréquence, qui est disposée entre les extrémités distales des bras de support (16, 18), **caractérisé en ce qu'**au moins l'un des bras de support (16, 18) présente, dans sa région d'extrémité distale (24), pour retenir des fragments tissulaires, une pièce de contact avec des tissus (26) disposée sur la paroi extérieure du bras de support (16, 18), coudée par rapport à l'axe longitudinal (LT) du bras de support (16, 18), la pièce de contact avec des tissus (26) étant réalisée et disposée pour retenir des fragments tissulaires (32) entre la pièce de contact avec des tissus (26) et l'extrémité distale d'un tube interne (28) et/ou l'extrémité distale d'un instrument de passage (30) d'un résectoscope (12).

2. Instrument à électrode (10) selon la revendication 1, **caractérisé en ce que** l'axe longitudinal (LG) de la pièce de contact avec des tissus (26) forme avec l'axe longitudinal (LT) du bras de support (16, 18) au moins en partie un angle (a) de 140° à 40°.

3. Instrument à électrode (10) selon l'une quelconque des revendications 1 et 2, **caractérisé en ce que** l'axe longitudinal (LG) de la pièce de contact avec des tissus (26) forme avec l'axe longitudinal (LT) du bras de support (16, 18) un angle (a) de 120° à 60°.

4. Instrument à électrode (10) selon l'une quelconque des revendications précédentes, **caractérisé en ce que** la pièce de contact avec des tissus (26) est constituée d'un matériau électriquement isolant.

5. Instrument à électrode (10) selon l'une quelconque des revendications précédentes, **caractérisé en ce que** la pièce de contact avec des tissus (26) est disposée dans une portion du bras de support (16, 18), qui est enveloppée par une gaine isolante (34) à isolation électrique.

6. Instrument à électrode (10) selon l'une quelconque des revendications précédentes, **caractérisé en ce que** la pièce de contact avec des tissus (26) s'étend à partir du bras de support (16, 18) sous forme cintrée en forme de cercle partiel à l'écart du bras de support (16, 18).

7. Instrument à électrode (10) selon l'une quelconque des revendications précédentes, **caractérisé en ce que** la pièce de contact avec des tissus (26) s'étend à partir du bras de support (16, 18) au moins jusque dans un plan sagittal (S) de l'instrument à électrode (10) qui s'étend à partir du bras de support (16, 18) sur 1/8 de la distance au deuxième bras de support (18, 16), de préférence sur 1/4 de la distance.

8. Instrument à électrode (10) selon l'une quelconque des revendications précédentes, **caractérisé en ce que** la pièce de contact avec des tissus (26) est réalisée et disposée de manière à s'enfoncer dans des fragments tissulaires (32).

9. Résectoscope (12) pour chirurgie endoscopique, comprenant un tube d'enveloppe (36), **caractérisé en ce qu'**à l'intérieur du tube d'enveloppe (36) est supporté de manière déplaçable longitudinalement un instrument à électrode (10) selon l'une quelconque des revendications 1 à 8.

10. Résectoscope (12) selon la revendication 9, **caractérisé en ce que** la pièce de contact avec des tissus (26) est disposée au moins en partie en position distale depuis un tube interne (28) disposé dans le tube d'enveloppe (36) et/ou un instrument de passage (30) disposé dans le tube d'enveloppe.

11. Résectoscope (12) selon la revendication 9, **caractérisé en ce que** la pièce de contact avec des tissus (26) est disposée au moins en partie en position proximale depuis l'électrode.
